# EUROPEAN PATENT APPLICATION

(11) **EP 4 066 884 A1**
(43) Date of publication of application: **05.10.2022**
(21) Application number: 21166518.7
(22) Date of filing: 01.04.2021
(51) Int. Cl.: A61N 1/375, A61N 1/372

(54) **IMPLANTABLE MEDICAL DEVICE COMPRISING AN ANCHORING DEVICE FOR ANCHORING ON AN ATRIAL WALL**

(71) Applicant: BIOTRONIK SE & Co. KG, 12359 Berlin (DE)
(72) Inventor: WHITTINGTON, R. Hollis, Portland, 97202 (US); HUGHES, Devan, Tualatin, 97026 (US); AUSTIN, Eric, Portland, 97221 (US); MUESSIG, Dirk, West Linn, 97068 (US); TAFF, Brian M., Portland, 97217 (US); MIDGETT, Madeline Anne, Portland, 97213 (US)
(74) Representative: Biotronik Corporate Services SE

(57) **Abstract**

An implantable medical device (1) comprises a housing (10) and an anchoring device (2) arranged on the housing (10) and being configured to anchor the implantable medical device (1) on an atrial wall (W) within an atrium (RA, LA) of a patient's heart. The anchoring device (2) comprises a piercing structure (20) configured to pierce the atrial wall (W) along a piercing direction (I) and a fixing structure (21) configured to reach into a pericardial space (S) beyond the atrial wall (W) for fixing the implantable medical device (1) on the atrial wall (W).

## Description

The present invention generally relates to an implantable medical device and a method for implanting an implantable medical device.

An implantable medical device of this kind may for example be an active implantable medical device (in short AIMD) which comprises electronic circuitry and an energy supply for performing a medical function within a patient's heart, such as a leadless pacemaker for implantation into the heart, for example in the right atrium of the heart.

In recent years, leadless pacemakers have received increasing attention. Leadless pacemakers, in contrast to pacemakers implanted subcutaneously using leads extending transvenously into the heart, avoid leads in that the pacemaker device itself is implanted into the heart, the pacemaker having the shape of a generally cylindrical capsule for implantation into cardiac tissue. Such leadless pacemakers exhibit the inherent advantage of not using leads, which can reduce risks for the patient involved with leads transvenously accessing the heart, such as the risk of pneumothorax, lead dislodgement, cardiac perforation, venous thrombosis and the like.

When placing an implantable medical device such as a leadless pacemaker device in the human heart, one challenge lies in designing an anchoring device allowing a fixation of the implantable medical device on cardiac tissue such that the implantable medical device is securely received and held on cardiac tissue. For this, the anchoring device on the one hand shall provide for a secure fastening of the implantable medical device on the cardiac tissue, on the other hand however shall avoid a damaging of cardiac tissue and structures within the cardiac tissue.

A typical anchoring device comprises anchoring members in the shape of tines which, when placing the implantable medical device on cardiac tissue, pierce the cardiac tissue and in this way provide for an anchoring of the implantable medical device on the cardiac tissue. For placing the implantable medical device on cardiac tissue, the implantable medical device for example is received within a sheathing device of a delivery catheter, the delivery catheter being used to access the human heart and to deliver the implantable medical device towards a location of interest by removing the sheathing device and by in this way placing the implantable medical device on cardiac tissue at the location of interest. Upon delivering the implantable medical device from the sheathing device the anchoring members engage with cardiac tissue to provide for a fastening of the implantable medical device on the cardiac tissue.

US 9,844,659 B2 describes an assembly of an implantable medical device comprising an anchoring device having a set of active fixation tines. The active fixation tines in the set are deployable from a spring-loaded position in which distal ends of the fixation tines point away from the implantable medical device to a hooked position in which the active fixation tines bend back towards the implantable medical device.

US 9,526,891 discloses an implantable pacemaker system including a housing having a proximal end and a distal end, an arrangement of anchoring members being placed on the distal end of the housing for providing for a fastening of the implantable pacemaker system on cardiac tissue.

US 8,700,181 discloses a leadless intra-cardiac medical device configured to be implanted entirely within a heart of a patient, the device including a housing configured to be securely attached to an interior wall portion of a chamber of the heart, and a stabilizing intra-cardiac device extension connected to the housing. The stabilizing intra-cardiac device extension may include a stabilizer arm or an appendage arm, or an elongated body or a loop member configured to be passively secured within the heart.

A typical implantable medical device such as a leadless pacemaker device is to be placed in a ventricle of the heart and is anchored on a ventricular wall. Tines of an anchoring device for this may pierce a portion of the ventricular wall and may hence fasten the implantable medical device on the ventricular wall, wherein a damage to the ventricular wall due its thickness and strength typically is limited. If an implantable medical device however shall be placed in the atrium, a fixation needs to be provided on an atrial wall, taking into account that the atrial wall generally has a reduced strength and delicate structure and hence is more susceptible to being damaged.

It is an objective of the present disclosure to provide an implantable medical device and a method for implanting an implantable medical device which allow for a reliable and secure fastening of an implantable medical device at a location of interest in an atrium of a patient's heart.

This object is addressed by an implantable medical device comprising the features of claim 1.

In one aspect, an implantable medical device comprises a housing and an anchoring device arranged on the housing and being configured to anchor the implantable medical device on an atrial wall within an atrium of a patient's heart. The anchoring device comprises a piercing structure configured to pierce the atrial wall along a piercing direction and a fixing structure configured to reach into a pericardial space beyond the atrial wall for fixing the implantable medical device on the atrial wall. The structure is likewise suitable for fixing the implantable medical device on the intra-atrial septum between the two atria, which is also considered and referred to as an atrial wall.

The implantable medical device shall be implanted into a patient's heart such that it comes to rest within an atrium of the heart, in particular the right atrium. For securing the implantable medical device within the atrium, herein, the anchoring device shall provide for an anchoring on an atrial wall bounding the atrium towards the outside, wherein due to the structure of the atrial wall, in particular its reduced strength and thickness in comparison e.g. to a ventricular wall, the anchoring device is shaped such that an anchoring may be provided despite the specific delicate structure of the atrial wall.

In particular, for providing for an anchoring on the atrial wall the anchoring device comprises a piercing structure which is used to pierce the atrial wall for providing a fixation on the atrial wall. Hence, when implanting the implantable medical device in the atrium, the piercing structure is brought into engagement with the atrial wall such that the piercing structure pierces through the atrial wall. With this, a fixing structure is guided through the atrial wall and, upon piercing the atrial wall with the piercing structure, comes to reach into a pericardial space or adjacent atrial cavity beyond the atrial wall such that a fixation of the implantable medical device on the atrial wall by means of the fixing structure is provided. In particular, due to the fixing structure reaching into the pericardial space or adjacent atrial cavity, it is prevented that the piercing structure may (unintentionally) be retracted from the atrial wall in a direction opposite to the piercing direction, such that in this way the implantable medical device is securely fixed on the atrial wall.

The implantable medical device in particular may be an active implantable medical device and for this may comprise electronic circuitry as well as an energy supply such that the implantable medical device, once implanted into the heart, may perform a control and/or monitoring function within the heart, such as a pacing function and/or a sensing function. The implantable medical device in particular may be a leadless pacemaker device configured for implantation into an atrium of the patient's heart, in particular the right atrium of the patient's heart.

In one embodiment, the implantable medical device comprises an electrode arranged on the housing. The implantable medical device may in particular have the shape of a longitudinal capsule having a distal end and a proximal end, the implantable medical device being placeable on tissue within the patient's heart with the distal end. The housing herein encapsulates electric and electronic components of the device in a fluid-tight manner, the electrode being placed distally on the housing, wherein on the proximal end the housing may for example comprise a coupling device which may be used to establish a coupling to a delivery catheter or the like. The electrode serves to transmit and/or receive electrical signals and for this, when the medical device is implanted into the heart, is in abutment with cardiac tissue, i.e. the atrial wall on which the implantable medical device is anchored by the anchoring device.

The anchoring device herein, in one embodiment, is arranged in the vicinity of the electrode, in particular on the distal end of the implantable medical device, such that the electrode and the anchoring device are substantially co-located on the housing, hence ensuring that the electrode is in abutment with cardiac tissue when the implantable medical device is anchored to cardiac tissue using the anchoring device.

In one embodiment, the piercing structure comprises a post for piercing the atrial wall. The post may be arranged on the housing such that it extends from the housing along the piercing direction, wherein the post carries the fixing structure such that the post may be introduced into the atrial wall with the fixing structure arranged thereon in order to guide the fixing structure through the atrial wall for placement beyond the atrial wall.

Alternatively, the piercing structure may be formed as a screw which may be screwed into the atrial wall in order to pierce the atrial wall for introducing the fixing structure into the space beyond the atrial wall.

In one embodiment, the piercing structure comprises a first end arranged on the housing and a second end remote from the housing, the fixing structure being arranged at said second end. The piercing structure hence, at its first end, is connected to the housing and extends from the housing to carry the fixing structure at its second end. When placing the implantable medical device on cardiac tissue the piercing structure with its second end comes to engage with the cardiac tissue and pierces the atrial wall, wherein the piercing structure is shaped such that the second end reaches through the atrial wall and comes to rest within the pericardial space or adjacent atrial cavity, such that also the fixing structure arranged on the second end is placed in that space and may act within the space for providing for a fixation of the implantable medical device on the atrial wall.

In one embodiment, the fixing structure extends from the second end of the piercing structure such that at least a portion of the fixing structure is located closer to the housing than said second end. The fixing structure hence points from the second end towards the housing of the implantable medical device, wherein the fixing structure may be elastic such that the fixing structure may provide for a tensioning to pull the housing of the implantable medical device with its distal end and an electrode arranged thereon towards the atrial wall in order to ensure a proper abutment of the housing and an electrode arranged thereon on the atrial wall from inside the atrium.

In one embodiment, the fixing structure is collapsible to assume a collapsed position when piercing the atrial wall with the piercing structure. The fixing structure hence is flexibly deformable and may assume its collapsed position when the piercing structure is engaged with the atrial wall. In the collapsed position the fixing structure is approached towards the piercing structure such that the fixing structure assumes a shape having a reduced radial extension in a plane perpendicular to the piercing direction, hence allowing to insert the fixing structure arranged on the piercing structure into and through the atrial wall for placing the fixing structure in the space beyond the atrial wall.

The fixing structure may be expandable from the collapsed position to assume an expanded position in which the radial extension of the fixing structure is widened in a plane perpendicular to the piercing direction with respect to the collapsed position. Once the pericardial space or adjacent atrial cavity is reached the fixing structure may be deployed to assume its expanded position, in which it is expanded to provide for a fixation of the implantable medical device on the atrial wall, the fixing structure hence ensuring that the anchoring device may not (unintentionally) be retracted from the atrial wall opposite to the piercing direction by forming a positive-locking connection in the pericardial space or adjacent atrial cavity and hence providing a locking of the anchoring device on the atrial wall.

In one embodiment, the fixing structure is self-expandable such that, once the anchoring device with the fixing structure has reached the pericardial space or adjacent atrial cavity, the fixing structure is self-deployed to assume the expanded position. Once the atrial wall is pierced by the piercing structure, hence, the fixing structure transitions from the collapsed position in which it is inserted through the atrial wall to the expanded position in which it provides for a locking of the implantable medical device on the atrial wall.

In the extended position the fixing structure may assume a preset shape. In particular, when being deformed from the expanded position towards the collapsed position the fixing structure is elastically tensioned such that it automatically resumes to the expanded position when it is free to expand, in particular once the pericardial space or adjacent atrial cavity is reached when piercing the atrial wall by means of the anchoring device.

In one embodiment, the fixing structure comprises at least one elastic strut which is fixed at one end to the piercing structure and extends from the piercing structure. The at least one elastic strut may for example be formed from a metal material, such as a shape memory alloy, for example a Nitinol material. A multiplicity of elastic struts herein may be arranged on the piercing structure, the elastic struts extending from the piercing structure in a generally radial direction, wherein the elastic struts are elastically deformable to assume a collapsed position for guiding the fixing structure through the atrial wall and to assume an expanded position for fixing the implantable medical device on the atrial wall.

In one embodiment, the at least one elastic strut points, with a directional vector component, in a direction opposite to the piercing direction. The at least one elastic strut may generally extend from the piercing structure in a radial direction, wherein the at least one elastic strut may form a sharp angle with the piercing direction and hence with a directional vector component points opposite to the piercing direction. A directional vector component herein is understood to correspond to a vector component of a vector indicating a direction of extension of the corresponding strut from the piercing structure, wherein the directional vector component is determined by the projection of the vector onto the axis of the piercing direction. By pointing in a direction opposite to the piercing direction the at least one elastic strut may abut with an epicardial side of the atrial wall in the pericardial space or adjacent atrial cavity and may provide for an elastic tensioning on and at the opposite side of the atrial wall, such that the implantable medical device is tensioned with respect to the atrial wall and is pulled into close abutment with the atrial wall, hence ensuring a proper abutment of an electrode arranged on a distal end of the housing of the implantable medical device.

In one embodiment, the at least one elastic strut is curved at a far end such that the at least one elastic strut, when viewed from the housing, comprises a convex curvature. The at least one elastic strut at one end is connected to the piercing structure and at a far end is free and remote from the piercing structure. At the far end the at least one elastic strut may be curved, such that the far end may not engage with the atrial wall at the opposite side within the pericardial space or adjacent atrial cavity, such that the at least one elastic strut may atraumatically rest on the atrial wall.

In one embodiment, the fixing structure comprises a flexible sheet arrangement which may be collapsible between a collapsed position in which the flexible sheet arrangement is approached towards the piercing structure and an expanded position in which the flexible sheet arrangement is radially expanded with respect to the piercing structure, to assume an umbrella-like shape for fixing the anchoring device at the opposite side of the atrial wall.

The flexible sheet arrangement may for example be made from a biocompatible material, such as a plastics material, for example a polymer material, or a silicone material.

The flexible sheet arrangement may be provided by a continuous sheet of material circumferentially extending about the piercing direction to radially extend from the piercing structure. The flexible sheet arrangement herein may be spanned by an arrangement of elastic struts, for example made of a metal material, in particular a shape memory alloy such as a Nitinol material, or made of a plastics material, for example a polymer material, or a silicone material. The elastic struts are connected to the piercing structure and provide for a tensioning on the sheet arrangement, the flexible sheet arrangement for example extending like a membrane across the elastic struts and being collapsible to approach the fixing structure towards the piercing structure.

In one embodiment, the fixing structure comprises a multiplicity of spikes, tines or barbs arranged on the piercing structure and providing for a fixation of the anchoring device on the atrial wall. Such spikes, tines or barbs may be provided in addition to elastic struts and/or a flexible sheet arrangement, or alternatively to elastic struts and a flexible sheet arrangement.

In another aspect, a method for implanting an implantable medical device into a patient's heart comprises: introducing the implantable medical device comprising a housing into an atrium of the patient's heart; and anchoring the implantable medical device on an atrial wall within the atrium using an anchoring device arranged on the housing; wherein said anchoring includes: piercing the atrial wall along a piercing direction using a piercing structure of the anchoring device such that a fixing structure reaches into a space beyond the atrial wall for fixing the implantable medical device on the atrial wall.

The advantages and advantageous embodiments described above for the implantable medical device equally apply also to the method, such that it shall be referred to the above in this respect.

The various features and advantages of the present invention may be more readily understood with reference to the following detailed description and the embodiments shown in the drawings. Herein,
- Fig. 1: shows a schematic view of the human heart;
- Fig. 2: shows a schematic drawing of an implantable medical device in the shape of a leadless pacemaker device, the implantable medical device having a housing and an anchoring device arranged thereon;
- Fig. 3: shows a schematic view of an embodiment of an implantable medical device with an anchoring device for fixing the implantable medical device on an atrial wall;
- Fig. 4: shows a schematic, enlarged view of the anchoring device of the implantable medical device;
- Fig. 5: shows a view of an anchoring device of an implantable medical device for anchoring the implantable medical device on an atrial wall, according to a another embodiment;
- Fig. 6: shows an implantable medical device with the anchoring device of Fig. 5, when introducing the anchoring device into an atrial wall;
- Fig. 7: shows the implantable medical device of Fig. 6, with the anchoring device being engaged with the atrial wall;
- Fig. 8: shows another embodiment of an implantable medical device with an anchoring device arranged thereon;
- Fig. 9: shows yet another embodiment of an implantable medical device with an anchoring device arranged thereon;
- Fig. 10: shows yet another embodiment of an implantable medical device with an anchoring device arranged thereon;
- Fig. 11: shows yet another embodiment of an implantable medical device with an anchoring device arranged thereon; and
- Fig. 12: shows an enlarged view of the anchoring device of the implantable medical device of Fig. 11.

Subsequently, embodiments shall be described in detail with reference to the drawings. In the drawings, like reference numerals designate like structural elements.

It is to be noted that the embodiments are not limiting for the present disclosure, but merely represent illustrative examples.

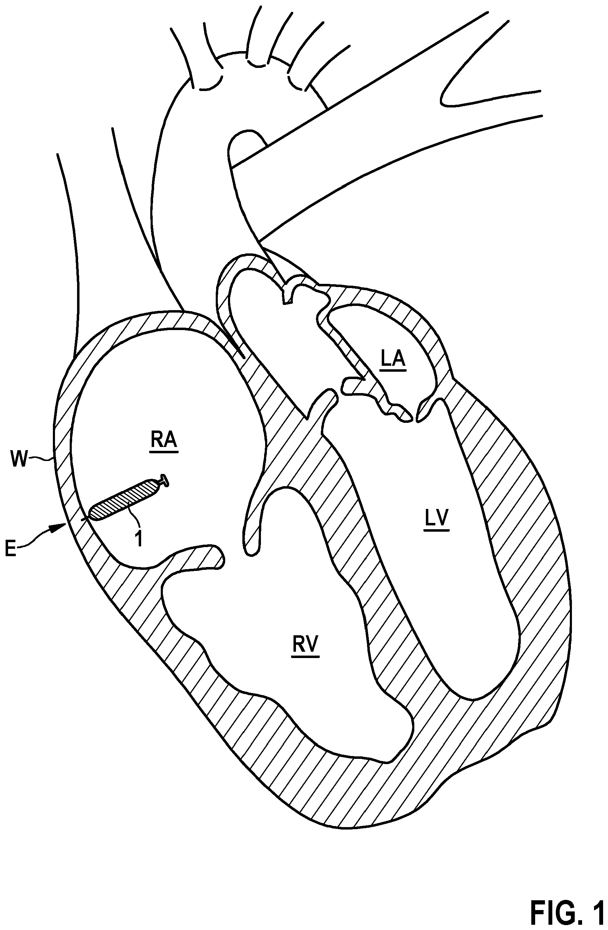

Fig. 1 shows, in a schematic drawing, a human heart comprising a right atrium RA, a right ventricle RV, a left atrium LA and a left ventricle LV, an implantable medical device 1 being implanted into the right atrium RA, the implantable medical device 1 for example having the shape of a leadless pacemaker device for providing a pacing of the heart's activity at the right atrium RA.

For implantation the implantable medical device 1 by means of a delivery system is placed in the right atrium RA and is delivered from the delivery system such that it comes to rest on intra-cardiac tissue on an atrial wall W and is fastened to the atrial wall W. The implantable medical device 1 could be fastened to any atrial wall including the interatrial septum.

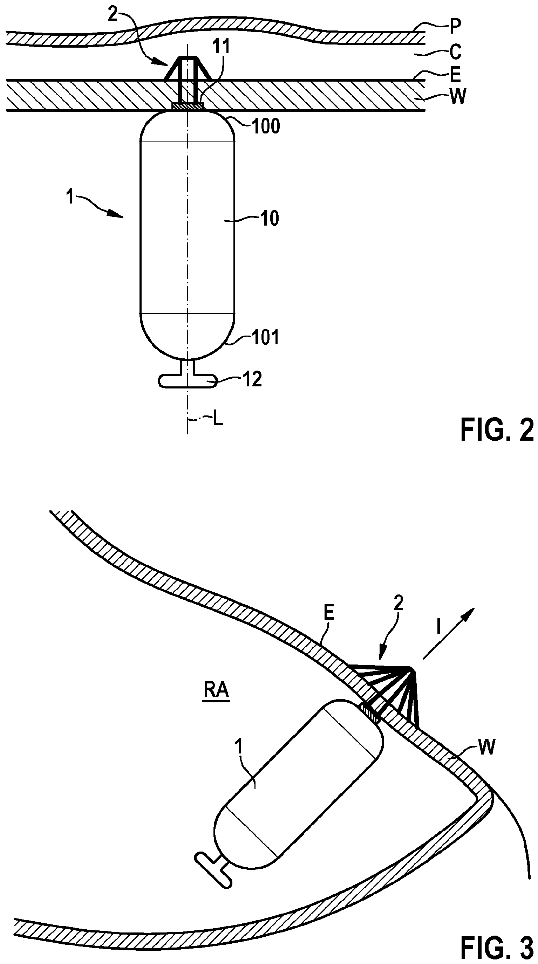

Fig. 2 shows, in a schematic drawing, an example of an implantable medical device 1 in the shape of a leadless pacemaker device, the implantable medical device 1 having a housing 10 forming a proximal end 101 and a distal end 100, the implantable medical device 1 being configured to be placed on an atrial wall W by means of its distal end 100. On the distal end 100, herein, an electrode 11 is placed which, potentially together with other electrodes of the implantable medical device 1, serves to provide for a pacing action in order to stimulate cardiac activity in a defined manner.

The implantable medical device 1 has the shape of a capsule extending longitudinally along a longitudinal axis L, the housing 100 encapsulating electrical and electronic components of the implantable medical device 1 therein in a fluid-tight manner. Whereas the distal end 100 with the electrode 11 arranged thereon is to be placed on and shall abut with intra-cardiac tissue for operation of the implantable medical device 1, the proximal end 101 carries a coupling device 12 which provides for a coupling of the implantable medical device 1 to a delivery device during implantation of the implantable medical device 1.

During implantation the implantable medical device 1 is to be placed on the atrial wall W and is to be fastened to the atrial wall W. For this, the implantable medical device 1 comprises an anchoring device 2, which shall provide for an anchoring to the atrial wall W and shall pierce the atrial wall W to ensure a proper fixation of the implantable medical device 1 on the atrial wall W.

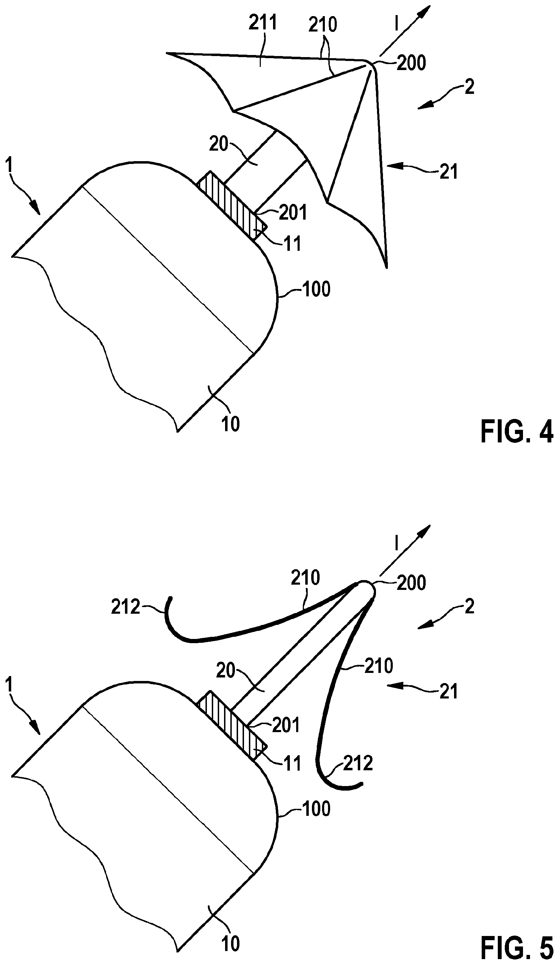

Referring now to Figs. 3 and 4, in one embodiment the anchoring device 2 comprises a piercing structure 20 in the shape of a post which for example is arranged on the distal end 100 of the housing 100 concentrically with respect to the electrode 11.

The piercing structure 20 in the shape of the post extends from the housing 10 of the implantable medical device 1 in a piercing direction I, is connected to the housing 10 at a first end 201 and on a second end 200 opposite the first end 201 carries a fixing structure 21, which in the embodiment of Figs. 3 and 4 has an umbrella-like shape in order to provide for a fixation of the implantable medical device 1 on the atrial wall W.

Herein, the piercing structure 20 serves to pierce the atrial wall W and to insert the fixing structure 21 through the atrial wall W such that the fixing structure 21 is introduced into a pericardial space S in between the atrial wall W and the pericardium P enclosing the heart, as illustrated in Fig. 2. The fixing structure 21 is collapsible such that, when being inserted through the atrial wall W by piercing the atrial wall W by means of the piercing structure 20, the fixing structure 21 assumes a collapsed state in which it is flexibly approached towards the piercing structure 20 and hence has a reduced radial extension with respect to the piercing structure 20. Once the fixing structure 21 has reached the pericardial space S, it may expand and hence assume an expanded position in which the fixing structure 21 is radially widened and hence provides for a fixation of the implantable medical device 1 on the atrial wall W in the sense of a positive-locking connection.

The fixing structure 21, in the embodiment of Figs. 3 and 4, may be formed by an arrangement of elastic struts 210 which are connected to and extend from the piercing structure 20. The struts 210 span a flexible sheet arrangement 211, which for example is formed from a sheet-like membrane extending in between and across the struts 210 to form an umbrella-like configuration.

The elastic struts 210 may for example be made from an elastic metal material, such as a shape memory alloy, for example a Nitinol material. The elastic struts 210 also may be made from a plastics material, such as a polymer material, or a silicone material.

The sheet arrangement 211 may be formed from a biocompatible plastics material, for example a polymer material, or a silicone material.

The fixing structure 21, in one embodiment, is self-expandable in that it automatically resumes from its collapsed position towards its expanded position once the fixing structure 21 reaches the pericardial space S and hence is free to deploy. In the extended position herein the fixing structure 21 may assume a preset shape and hence may provide for a locking on an epicardial side E of the atrial wall W, i.e. outside of the atrial wall W.

Because the fixing structure 21 is introduced transmurally through the atrial wall W into the pericardial space S beyond the atrial wall W, a secure fixation of the implantable medical device 1 on the atrial wall W may be provided, while requiring only a small piercing hole in the atrial wall W due to the piercing by the piercing structure 20. Once the fixing structure 21 expands to assume the expanded position, the implantable medical device 1 may be tensioned with respect to the atrial wall W, such that the implantable medical device 1 with its distal end 100 and the electrode 11 arranged thereon is pulled into tight contact with the atrial wall W to ensure proper operation of the implantable medical device 1. A tight contact of the implantable medical device 1 with the atrial wall W herein may also help to avoid a blood loss and to encourage encapsulation of the implantable medical device 1 on the atrial wall W.

Referring now to Figs. 5 to 7, in another embodiment the fixing structure 21 may be formed by an arrangement of elastic struts 210, without a flexible sheet arrangement spanning across the struts 210. The elastic struts 210 are arranged on a piercing structure 20 in the shape of a post which extends from the housing 10 of the implantable medical device 1 along the piercing direction I. The struts 210 may be formed from an elastic metal material, such as a shape memory alloy, for example a Nitinol material, or may be made from a biocompatible plastics material, for example a polymer material, or a silicone material.

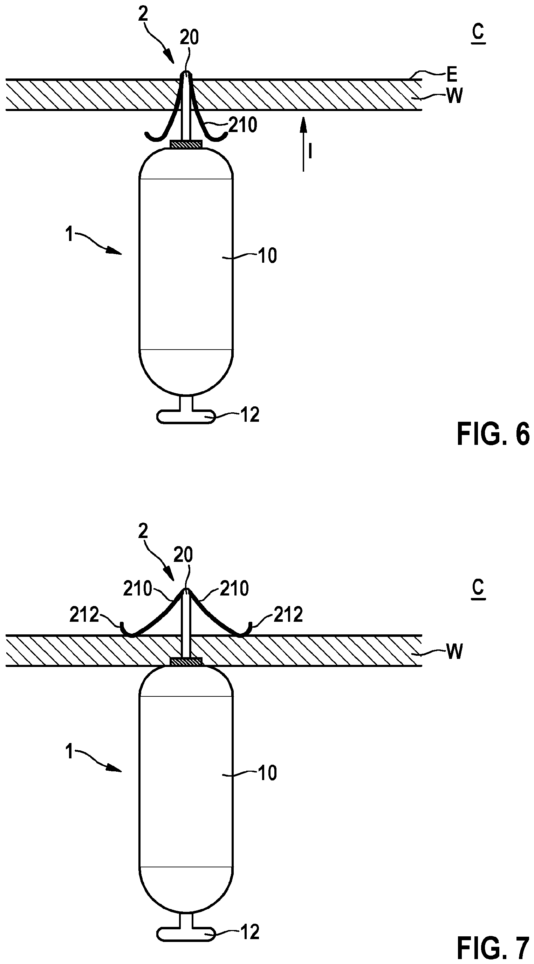

When being introduced through the atrial wall W, as shown in Fig. 6, the struts 210 may be collapsed towards the piercing structure 20 such that the fixing structure 21 may easily be pushed through the atrial wall W in the piercing direction I together with the piercing structure 20. Once the struts 210 have reached the pericardial space S, as shown in Fig. 7, the struts 210 may transition towards their expanded position and may abut with an epicardial side E of the atrial wall W to anchor the implantable medical device 1 on the atrial wall W.

In the embodiment of Figs. 5 to 7 ends 212 of the struts 210 are angled away from the atrial wall W in that the struts 210 in the region of the ends 212 are shaped to have a convex curvature, when viewed from the side of the housing 10. Hence, the struts 210 may abut with the atrial wall W in an atraumatic fashion, as this is visible from Fig. 7.

In another embodiment, the struts 210 may be angled, at their ends 212, towards the atrial wall W such that the struts 210 assume a concave curvature when viewed from the side of the housing 10. The struts 210 hence may engage with the atrial wall W at the epicardial side E with the ends 212.

In the embodiments of Figs. 3 and 4 and of Figs. 5 to 7, the struts 210 are angled with respect to the piercing structure 20 in the shape of the longitudinal post such that a sharp angle is formed in between the struts 210 and the piercing direction I. The struts 210 herein, with a directional vector component, point towards the atrial wall W, the directional vector component corresponding to a projection of a general vector of extension of the struts 210 with respect to the piercing structure 20 onto the piercing direction I.

Due to the angle of extension of the fixing structure 21 with respect to the piercing structure 20 the fixing structure 21 may provide for a tensioning of the implantable medical device 1 with respect to the atrial wall W, allowing hence for a proper fixation and in addition a tensioning of the implantable medical device 1 on the atrial wall W.

In the embodiments of Figs. 3 and 4 and of Figs. 5 to 7 the fixing structure 21 may be formed strong enough such that a retraction of the anchoring device 21 opposite the piercing direction I is effectively prevented. Alternatively, the fixing structure 21, in particular the struts 210, may be formed to be elastically bendable such that they may allow for a retraction of the anchoring device 2 opposite the piercing direction I, by applying a sufficient retracting force to the housing 10, to remove the implantable medical device 1 from the atrial wall W.

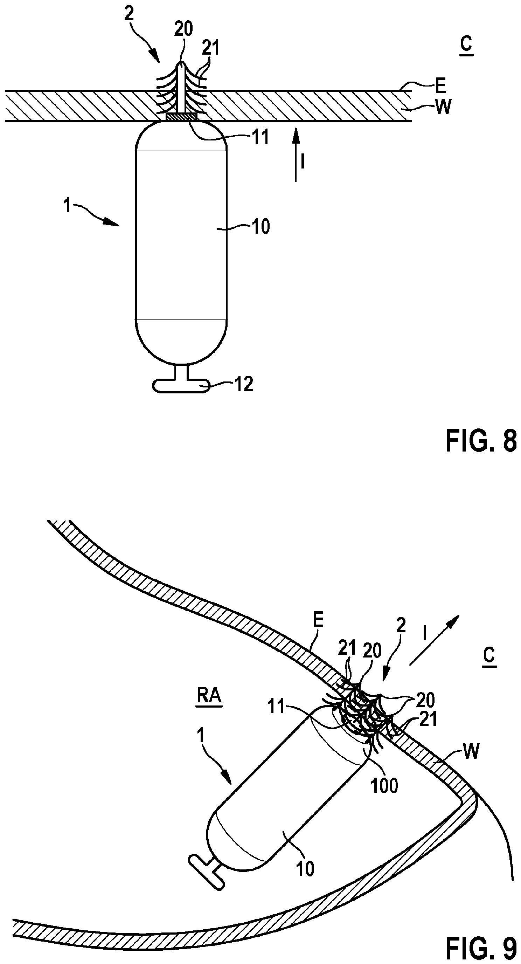

Referring now to Fig. 8, in another embodiment the fixing structure 21 may be formed by an arrangement of spikes, tines or barbs. A multiplicity of spikes, tines or barbs herein may be distributed on the piercing structure 20 around the piercing direction I and also along the axial length of the piercing structure 20 along the piercing direction I, wherein at least a portion of the spikes, tines or barbs are placed inside the pericardial space S or adjacent atrial cavity upon insertion of the anchoring device 2 into the atrial wall W.

In another embodiment, shown in Fig. 9, multiple of the anchoring devices 2 as shown in Fig. 8 may be provided on the distal end 100 of the housing 10, wherein the anchoring devices 2 may for example be arranged around a central electrode 11 placed on the distal end 100 of the housing 10.

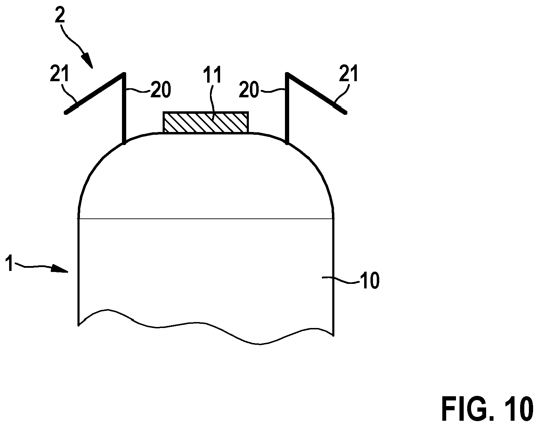

Referring now to Fig. 10, in yet another embodiment anchoring devices 2 having the shape of hooks may be provided, each anchoring device 2 comprising a piercing structure 20 in the shape of a post and a fixing structure 21 in the shape of a strut arranged at an angle with respect to the piercing structure 20 to reach into the space S beyond the atrial wall W. Multiple anchoring devices 2 herein may be placed around a central electrode 11, wherein for each anchoring device 2 the fixing structure 21 in the shape of the strut may be placed radially outwards with respect to the piercing structure 20.

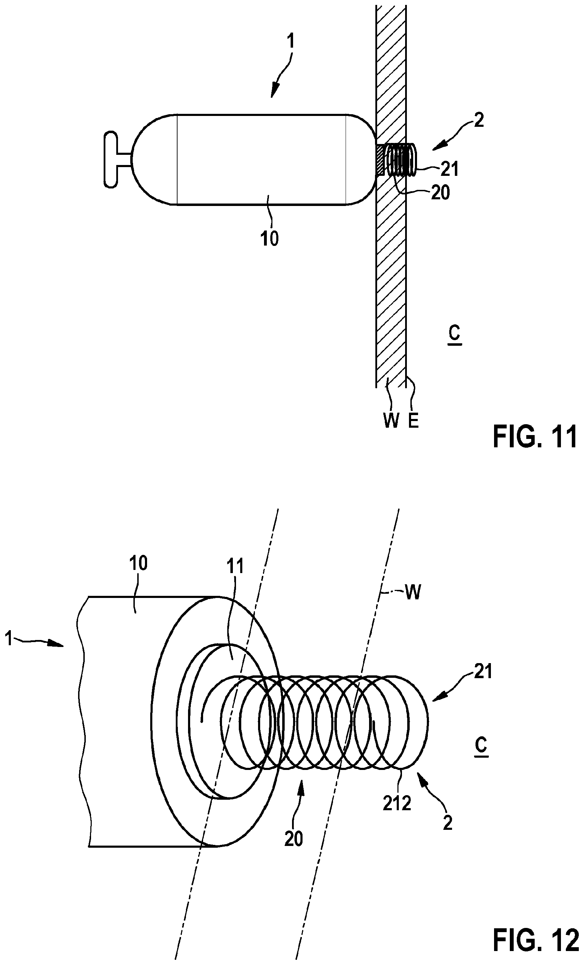

Referring now to Figs. 11 and 12, the anchoring device 2, in one embodiment, comprises a piercing structure 20 in the shape of a screw, which is formed to engage with and being screwed through the atrial wall W to reach into the space S for providing for a fixation of the implantable medical device 1 on the atrial wall W.

A fixing structure 21 herein may be formed on the piercing structure 20 by means of one or multiple turns of the screw forming the piercing structure 20, the turns being placed within the space S and having for example an increased diameter with respect to the turns of the piercing structure 20 to be placed and received within the atrial wall W. An end 212 of the fixing structure 21 herein may be shaped to be atraumatic in that it for example is angled away from the atrial wall W.

The fixing structure 21 in the embodiment of Figs. 11 and 12, alternatively, may be formed by another structure, such as an umbrella-like structure as shown in the embodiment of Figs. 3 and 4 or a strut arrangement as shown in the embodiment of Figs. 5 to 7.

An anchoring device as described herein may provide for an easy deployment, which may involve a single pushing motion through an atrial wall.

A correct deployment herein may easily be verified by visualization using for example a suitable imaging technique, such as an X-ray technique, the deployment being recognizable by a proper expansion of the fixing structure.

An anchoring device as described herein may be compatible with thin and delicate tissue structures, such as the atrial wall, while providing a secure fixation with the possibility of a tensioning of the implantable medical device on the tissue.

### LIST OF REFERENCE NUMERALS

- 1: Leadless pacemaker device
- 10: Housing
- 100: Distal end
- 101: Proximal end
- 11: Electrode
- 12: Coupling device
- 2: Anchoring device
- 20: Piercing structure (post)
- 200, 201: End
- 21: Fixing structure
- 210: Strut
- 211: Sheet arrangement
- 212: Far end
- S: Pericardial or adjacent atrial space
- E: Epicardial side
- I: Piercing direction
- L: Longitudinal axis
- LA: Left atrium
- LV: Left ventricle
- P: Pericardium
- RA: Right atrium
- RV: Right ventricle
- W: Atrial wall (including septum)

## Claims

1. An implantable medical device (1), comprising:
a housing (10); and
an anchoring device (2) arranged on the housing (10) and being configured to anchor the implantable medical device (1) on an atrial wall (W) within an atrium (RA, LA) of a patient's heart, the anchoring device (2) comprising a piercing structure (20) configured to pierce the atrial wall (W) along a piercing direction (I) and a fixing structure (21) configured to reach into a pericardial or adjacent atrial space (S) beyond the atrial wall (W) for fixing the implantable medical device (1) on the atrial wall (W).

2. The implantable medical device (1) according to claim 1, comprising an electrode (11) being arranged on the housing (10), the electrode (11) being configured to abut the atrial wall (W) for at least one of emitting an electrical signal and receiving an electrical signal.

3. The implantable medical device (1) according to claim 2, wherein the anchoring device (2) is arranged on the housing (10) in the vicinity of the electrode (11).

4. The implantable medical device (1) according to one of claims 1 to 3, wherein the piercing structure (20) comprises a post for piercing the atrial wall (W).

5. The implantable medical device (1) according to one of the preceding claims, wherein the piercing structure (20) comprises a first end (201) arranged on the housing (10) and a second end (200) remote from the housing (10), the fixing structure (21) being arranged at said second end (200).

6. The implantable medical device (1) according to claim 5, wherein the fixing structure (21) extends from said second end (200) of the piercing structure (20) such that at least a portion of the fixing structure (21) is located closer to the housing (10) than said second end (200).

7. The implantable medical device (1) according to one of the preceding claims, wherein the fixing structure (21) is collapsible to assume a collapsed position when piercing the atrial wall (W) with the piercing structure (20), the fixing structure (21) being approached towards the piercing structure (20) in the collapsed position and being expandable from the collapsed position to assume an expanded position in which the radial expansion of the fixing structure (21) in a plane perpendicular to said piercing direction (I) is widened with respect to the collapsed position.

8. The implantable medical device (1) according to claim 7, wherein the fixing structure (21) is self-expandable and in the expanded position assumes a pre-set shape.

9. The implantable medical device (1) according to one of the preceding claims, wherein the fixing structure (21) comprises at least one elastic strut (210) being fixed at one end to the piercing structure (20) and extending from the piercing structure (20).

10. The implantable medical device (1) according to claim 9, wherein the at least one elastic strut (210) extends from the piercing structure (20) such that the at least one elastic strut (210) points, with a directional vector component, in a direction opposite to the piercing direction (I).

11. The implantable medical device (1) according to claim 9 or 10, wherein the at least one elastic strut (210) is curved at a far end (212) such that the at least one elastic strut (210), when viewed from the housing (10), comprises a convex curvature.

12. The implantable medical device (1) according to one of the preceding claims, wherein the fixing structure (21) comprises a flexible sheet arrangement (211).

13. The implantable medical device (1) according to claim 12, wherein the flexible sheet arrangement (211) is spanned by a multiplicity of elastic struts (210) connected to the piercing structure (20).

14. The implantable medical device (1) according to one of the preceding claims, wherein the fixing structure (21) comprises a multiplicity of spikes, tines or barbs arranged on the piercing structure (20).

15. A method for implanting an implantable medical device (1) into a patient's heart, comprising:
introducing the implantable medical device (1) comprising a housing (10) into an atrium of the patient's heart; and
anchoring the implantable medical device (1) on an atrial wall (W) within the atrium (RA, LA) using an anchoring device (2) arranged on the housing (10);
wherein said anchoring includes: piercing the atrial wall (W) along a piercing direction (I) using a piercing structure (20) of the anchoring device (2) such that a fixing structure (21) reaches into a pericardial space (S) or adjacent atrial cavity beyond the atrial wall (W) for fixing the implantable medical device (1) on the atrial wall (W).
